(19) Europäisches Patentamt
European Patent Office
Office européen des brevets

(11) Publication number: **0 341 213 B1**

(12) # EUROPEAN PATENT SPECIFICATION

(45) Date of publication of patent specification: **22.02.95**

(51) Int. Cl.[6]: **C07D 495/14**, C07D 491/14, A61K 31/435, C07F 7/10, //(C07D495/14,333:00,235:00, 221:00),(C07D491/14,307:00, 235:00,221:00)

(21) Application number: **89810333.8**

(22) Date of filing: **01.05.89**

(54) **5-Aryl-substituted-2,3-dihydro-imidazo [1,2-a]furo[3,2-c]- or thieno[2,3-c]pyridines.**

(30) Priority: **05.05.88 US 190566**

(43) Date of publication of application:
**08.11.89 Bulletin 89/45**

(45) Publication of the grant of the patent:
**22.02.95 Bulletin 95/08**

(84) Designated Contracting States:
**AT BE CH DE ES FR GB GR IT LI LU NL SE**

(56) References cited:
**WO-A-88/00587**

(73) Proprietor: **SANDOZ AG**
**Lichtstrasse 35**
**CH-4002 Basel (CH)**
(84) Designated Contracting States:
**BE CH ES FR GB GR IT LI LU NL SE**

(73) Proprietor: **SANDOZ-PATENT-GMBH**
**Humboldtstrasse 3**
**D-79539 Lörrach (DE)**
(84) Designated Contracting States:
**DE**

(73) Proprietor: **SANDOZ-ERFINDUNGEN Verwaltungsgesellschaft m.b.H.**
**Brunner Strasse 59**
**A-1230 Wien (AT)**
(84) Designated Contracting States:
**AT**

(72) Inventor: **Cheon, Seung Hoon**
**120 Carteret Street**
**Glen Ridge, N.J. 07028 (US)**
Inventor: **Houlihan, William Joseph**
**15 Raynold Road**
**Mountain Lakes, N.J. (US)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

## Description

The present invention relates to 5-aryl-sustituted-2,3-dihydro-imidazo[1,2-a]thieno pyridines and to their use as platelet activating factor (PAF) receptor antagonists and anti-tumor agents. The invention also relates to pharmaceutical compositions containing the afore-mentioned compounds as an active ingredient thereof and to the method of using such compositions for inhibiting PAF-mediated bronchoconstriction and extravasation, for controlling hyperreactive airways induced by PAF or allergen, for protection against endotoxin-induced hypotension and death and in treating tumors.

USP 3,887,566 discloses 2,3-dihydroimidazo-isoquinolines exhibiting analgesic, anti-inflammatory, anti-bacterial, anti-viral and cardiovascular properties. USP 4,100,165 discloses 5-hydroxy-2,3,5,6-tetrahydrofuran imidazo[2,1-a]isoquinolines containing a pyridyl-, thienyl- or furyl ring in the 5-position, which compounds are useful as anorexics and anti-depressants. USP 4,101,553 discloses 5-hydroxy-2,3,5,6-tetrahydrofuran imidazo[2,1-a]isoquinolines containing an optionally substituted aryl group in the 5-position, which compounds are useful as anorexics and anti-depressants. WO 88/587 discloses 5-aryl- or 5-heteroaryl-substituted imidazo-[2,1-a]isoquinolines exhibiting PAF-receptor antagonistic and anti-tumor activity.

The present invention concerns compounds of formula I

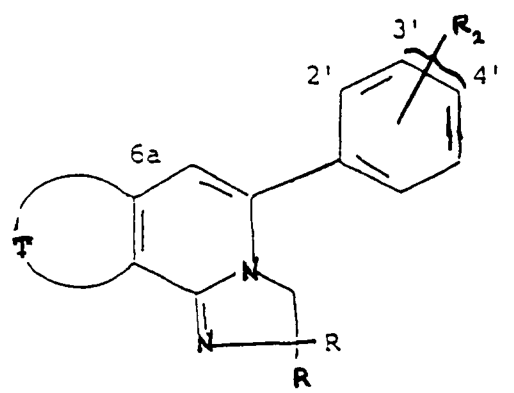

I

wherein

each R is, independently, hydrogen or methyl;

T is

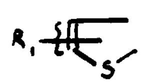

(d)

where $R_1$ is hydrogen or methyl; and

$R_2$ is a group of the formula

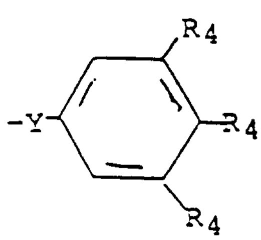

,

where

Y is $-(CH_2)_{1-3}$, $-OCH_2-$ or $-OCH_2CH_2-$, and each $R_4$, independently, is hydrogen or

$C_{1-3}$alkoxy;

in free base form or in acid addition salt form.

It will be appreciated that $R_2$ in formula I is attached to the phenyl ring in the 3' or 4' position. It preferably is attached in the 4' position.

Each R preferably is hydrogen.

$R_1$ preferably is hydrogen. When it is methyl, it preferably is attached to the carbon atom at the position adjacent to the sulfur atom.

A group of compounds of formula I is the compounds of formula I':

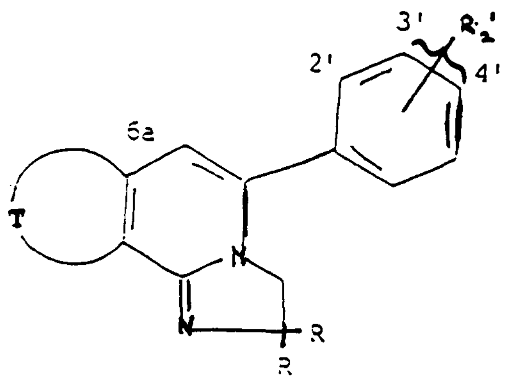

I'

where

$R_2'$ is a group of the formula

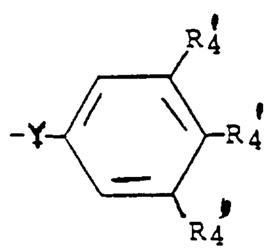

,

where

Y is as defined above and the $R_4'$ 's are the same and are $C_{1-3}$alkoxy;

and the R 's and T are as defined above,

in free base form or in acid addition salt form.

A further group of compounds of formula I is the compounds of formula I":

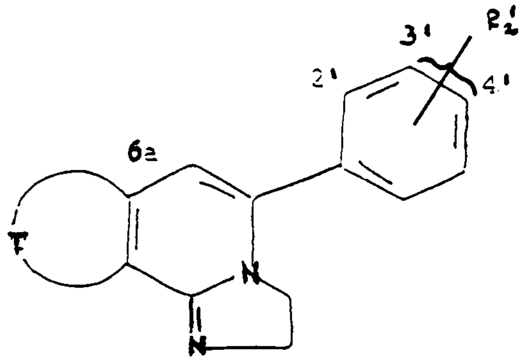

I"

wherein
T and $R_2$' are as defined above,
in free base form or in acid addition salt form.

A further group of compound of formula I is the compounds of formula Is:

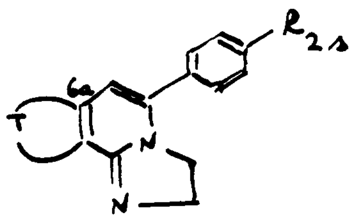

Is

wherein
T is as defined above and
$R_{2s}$ is a group of formula

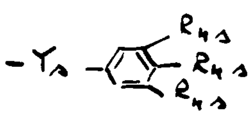

wherein $Y_s$ is $-(CH_2)_{1-3}-$ and each $R_{4s}$ independently is $C_{1-3}$alkoxy,
in free base form or in acid addition salt form.

A compound of formula I may be prepared by a process comprising dehydrating a corresponding compound of formula V:

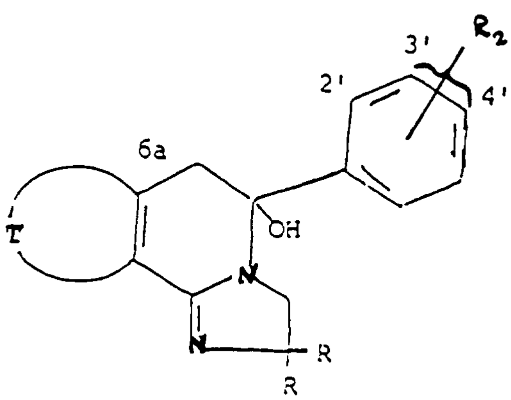

V

where the R's, T and $R_2$ are as defined above,
and recovering the resultant compound of formula I in free base form or in acid addition salt form.

The above process may be effected in accordance with known methods. The dehydration preferably is effected in an inert, organic solvent in the presence of an acid catalyst, which can be any mineral acid such as hydrochloric acid, sulfuric acid, phosphoric acid or an organic acid, e.g., an alkylcarboxylic acid such as acetic acid, an arylcarboxylic acid such as benzoic acid, an alkylsulfonic acid such as methanesulfonic acidor an arylsulfonic acid such as p-toluenesulfonic acid. The preferred acid catalysts are alkylcarboxylic acids, more preferably, acetic acid, and arylsulfonic acids, more preferably, p-toluenesulfonic acid. The inert solvent is usually an aliphatic hydrocarbon such as hexane, heptane, an aromatic hydrocarbon such as benzone, toluene, a chlorinated hydrocarbon such as chloroform, methylene chloride, an aliphatic ether such as diethyl ether, a cyclic ether such as tetrahydrofuran, or an excess of a liquid acid catalyst, preferably acetic acid, or p-toluenesulfonic acid may serve as the solvent.
The temperature preferably is in the range of between 35° and 200°C, especially between about 75° and about 120°C.

The starting materials can be prepared in accordance with known procedures, e.g. according to the following reaction scheme:

REACTION A

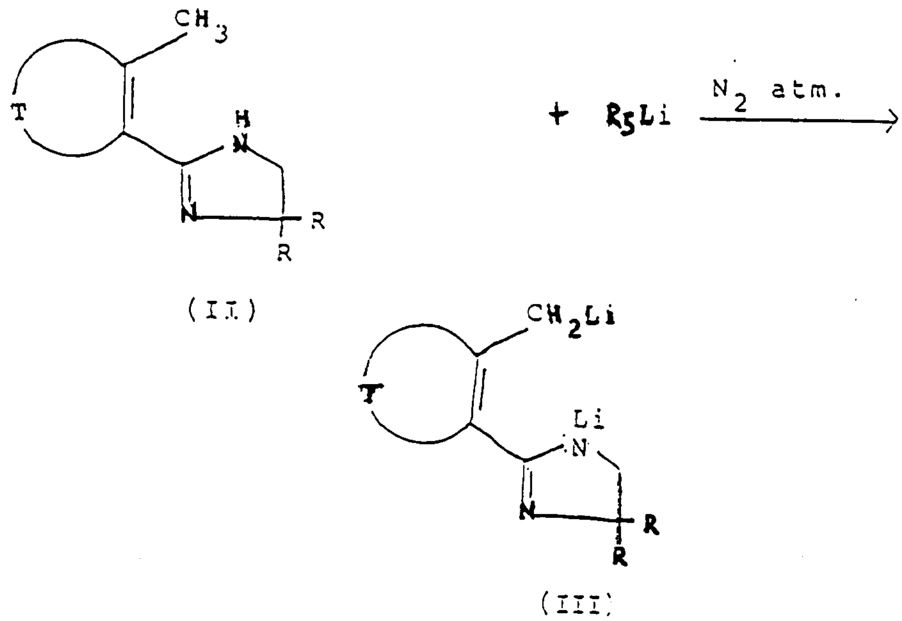


(II)

(III)

where $R_5$ is straight or branched chain $C_{1-4}$alkyl, T is (a), (b), (c) or (d) as defined above and the R 's are as defined above.

REACTION B

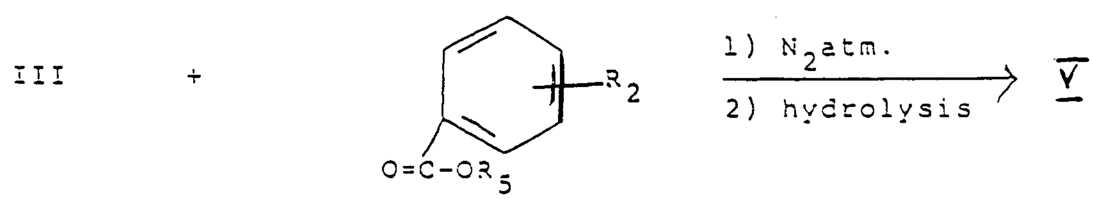


(IV)

where $R_5$, T, $R_2$ and the R 's are as defined above.

Reaction A is generally carried out in an inert, organic solvent. An inert atmosphere of nitrogen is preferred. A complexing or activating agent for the lithium compound, e.g. tetramethylethylenediamine is optionally added. The temperature is in the range of between about 0° and about -78°C, preferably between about -50° and about -78°C.

Reaction B is also usually effected under a nitrogen atmosphere in the first step. Preferably an aliphatic hydrocarbon is used as the solvent, or an aliphatic or cyclic ether. The temperature is in the range of from about -78° to 25°C, preferably about -78° to about 20°C. The adduct formed is then hydrolyzed in a second step, e.g. employing water, dilute mineral acid ammonium chloride solution

The starting materials of formula II and IV are either known and/or may be obtained analogously to known methods in conventional manner.

Final products and intermediates may be isolated and purified in conventional manner. Intermediates, where appropriate, may be employed directly in the following reaction step without purification.

As is evident to those skilled in the art, the compounds of formula I may exist in racemic or enantiomeric form and the invention is intended to cover all forms. Enantiomeric forms may be recovered in conventional manner, e.g. by resolution of end or intermediate products or by employing optically active starting materials.

Examples of pharmaceutically acceptable acid addition salts include those with mineral acids, e.g. hydrochloric, hydrobromic, phosphoric and sulfuric acids, and organic acids, e.g. tartaric, acetic, citric, malic, maleic, methanesulfonic and gluconic acids, which may be prepared in conventional manner.

The compounds of formula I in free base form or in pharmaceutically acceptable acid addition salt form, hereinafter referred to as "the compounds", are indicated for use as platelet activating factor (PAF) receptor antagonists, as indicated by their ability to inhibit specific binding of [$^3$H]-PAF to platelets according to the Human Platelet PAF Receptor Assay test (test A) as described in WO 88/587.

Moreover, in view of their activity as PAF receptor antagonists, the compounds are indicated for use as inhibitors of PAF-mediated bronchoconstriction, which property is evaluated by the PAF-induced Pulmonary Inflation Pressure (PIP) Increase test (test B) as described in WO 88/587, to generate an $ED_{50}$ (dose needed to effect a 50 % response).

Furthermore, the compounds are indicated for use as inhibitors of PAF-mediated extravasation (the extrusion of plasma from the lumen of the blood vessels into the vessel wall and surrounding tissues) measured as a function of hemoconcentration according to the PAF-induced Extravasation test (test C) as described in WO 88/587. From the values obtained an $ED_{50}$ is generated.

Still further, the compounds are indicated for use in controlling hyperreactive airways induced by PAF or allergen, which property can be measured in accordance with the following procedure (test D):

Male Hartley guinea pigs weighing 250gm are sensitized to ovalbumin by aerosol inhalation exposure. The test animals are then subsequently rechallenged with ovalbumin aerosol repeatedly (3 to 6 times) over a period of two to three weeks. Airway reactivity is assessed by an acetylcholine dose response curve at times (1 to 3 days) after the last ovalbumin exposure. The test compound is assessed for its ability to control hyperreactivity airways by administering it orally with a gavage tube in an acceptable vehicle prior to each ovalbumin antigen exposure.

Yet still further, the compounds are indicated for use in protecting against endotoxin-induced hypotension, which property can be measured according to the following procedure (test E):

Male Sprague-Dawley rats weighing between 250 and 270gm are anesthetized with sodium pentobarbital (50mg/kg i.p.) and the left common carotid artery is cannulated (PE-50 tubing) and connected to a P50 pressure transducer. Mean arterial pressures and diastolic and systolic measurements are recorded using a Gould 2400S physiograph. Blood flow of the mesenteric artery is measured on a calibrated electromagnetic flowmeter probe. Blood is collected via the femoral artery into heparinized capillary tubes and centrifuged to determine hematocrit values.

Endotoxin from E.coli serotype 0111:$B_4$ is prepared fresh daily and administered by i.v. injection to the test animals in tris-Tyrode's buffer over a 1 to 50 mg/kg dosage range to establish a dose-response profile. The administration of endotoxin at 15mg/kg i.v. produced a 54±8% decrease in mean arterial pressure and a corresponding 80% decrease in mesenteric artery blood flow. The test compound is assessed for its ability to protect against endotoxin-induced hypotension by administering it intravenously after endotoxin administration and measuring the recovery of blood pressure and mesenteric artery blood flow. The $ED_{50}$ value of the test compound is determined using linear regression fitting of inhibition profiles from 5 to 6 doses (3 animals per dose).

Yet even still further, the compounds are indicated for use in protecting against endotoxin-induced death, which property can be measured according to the following procedure (test F):

Healthy male BALB/c mice weighing between 24 and 27g. are allowed to acclimate for 1 week with access to food and water. The test animals are then placed in a ventilated plexiglass restrainer that allows access to the tails. After the tails are allowed to immerse in warm water ( 38 °C) for 30 seconds, endotoxin from E. coli serotype 0111:$B_4$ is administered in a single injection at 2ml/kg body weight to produce lethality at the desired effect of LD 70-90. The test compound is assessed for its ability to protect against endotoxin-induced death by administering it orally in a single bolus at a volume of 1ml/kg body weight. Each treatment group consists of 7 to 10 test animals, every dosage is considered as a separate group and control groups are dosed with vehicles (water, tris-Tyrodes's buffer, 1% CMC, etc) only. The percent mortality (or survival) is expressed by the number of deaths (or survivors) within the observation period. Values obtained are mean and standard error of mean of a single treatment which represents multiple days results for reproducibility. The $ED_{50}$ value of the test compound is determined using a Students t test (2

tail) for significance.

The compounds are thus indicated for use in treating PAF-mediated bronchoconstriction and extravasation, for controlling hyperreactive airways induced by PAF or allergen and protecting against endotoxin-induced hypotension and death. An indicated daily dosage is from about 100 mg to about 2000 mg, preferably from about 10 mg to about 350 mg. A typical oral dosage is 50 mg or 100 mg, two or three times a day.

Further, the compounds are indicated for use as anti-tumor agents and, particularly, in inhibiting the growth of lymphomas, sarcomas, myelomas and leukemia cell lines. The ability of the compounds in treating tumors can be measured by the Tumor Cell Cytotoxicity test (TCC test) as described in WO 88/587.

The anti-tumor activity may also be demonstrated employing the Influence on Cytotoxicity of ET-18-$OCH_3$ test (IC-ET test) as described in WO 88/587 or the test described as test F in WO 88/587.

The compounds are thus further indicated for use in inhibiting tumors. An indicated daily dosage is from about 500 mg to about 2000 mg, preferably from about 1000 mg to about 1500 mg. A typical oral dosage is about 400 mg, two to three times a day, or about 20 mg/kg intravenously over a 24 hour period.

The compounds may be combined with one or more pharmaceutically acceptable carriers and, optionally, one or more other conventional pharmaceutical adjuvants and administered orally in the form of tablets, dispersible powders, granules, capsules, elixirs, suspensions, etc., or parenterally in the form of sterile injectable solutions or suspensions. The compositions may be prepared by conventional means.

Pharmaceutical compositions comprising a compound of formula I in free base form or in pharmaceutically acceptable acid addition salt form are also part of the invention.

Examples of pharmaceutical compositions are:

| Ingredients | Weight (mg) | |
|---|---|---|
| | tablet | capsule |
| compound of formula I, e.g., the compound of Example 1 in hydrochloride acid addition salt form | 50 | 50 |
| tragacanth | 10 | - |
| lactose (spray-dried) | 212.5 | 100 |
| corn starch | 15 | - |
| talcum | 10 | - |
| magnesium stearate | 2.5 | - |
| Total | 300.0 | 100 |

| Ingredients | Weight (mg) | |
|---|---|---|
| | tablet | capsule |
| compounds of formula I, e.g., the compound of Example 1 in hydrochloride acid addition salt form | 400 | 400 |
| tragacanth | 10 | - |
| lactose (spray-dried) | 197.5 | 250 |
| corn starch | 25 | - |
| talcum | 15 | - |
| magnesium stearate | 2.5 | - |
| Total | 650.0 | 650 |

| Ingredients | Weight (mg) | |
|---|---|---|
| | sterile injectable suspension | oral liquid suspension |
| compound of formula I, e.g., the compound of Example 1 in hydrochloride acid addition salt form | 5 | 3 |
| sodium carboxymethylcellulose U.S.P. | 1 | 8 |
| methyl cellulose | 0.3 | - |
| polyvinylpyrrolidone | 2.7 | - |
| lecithin | 1.5 | - |
| benzyl alcohol | 0.01 | - |
| magnesium aluminum silicate | - | 25 |
| flavor | - | q.s. |
| color | - | q.s. |
| methyl paraben, U.S.P. | - | 3 |
| propyl paraben, U.S.P. | - | 0.7 |
| polysorbate 80 (e.g. Tween 80), U.S.P. | - | 5 |
| sorbitol solution, 70%, U.S.P. | - | 1450 |
| buffer agent to adjust pH for desired stability | q.s. for injection | q.s. |
| water | q.s. to 1 ml | q.s. to 5 ml |

The preferred pharmaceutical compositions from the standpoint of preparation and ease of administration are solid compositions, particularly liquid or hard-filled capsules and tablets containing from about 10 mg to about 100 mg of the active ingredient concerning the PAF inhibition use and from about 350 mg to about 450 mg of the active ingredient with respect to tumor inhibition.

The compound of Example 1, especially the compound of Example 1 in hydrochloride acid addition salt form, is preferred.

The following results were obtained in the above tests:

Compound of Example 1 (hydrochloride):

Test A: $IC_{50}$ = 0.01 μM
Test B: $ED_{50}$ = 1.0 mg/kg p.o.
Test C: $ED_{50}$ = 1.0 mg/kg p.o.

The invention also includes a method for the preparation of a pharmaceutical composition comprising mixing a compound of formula I in free base form or in pharmaceutically acceptable acid addition salt form with a pharmaceutically acceptable carrier or diluent.

It also includes the use of such a compound for the preparation of a pharmaceutical composition comprising a compound of formula I in free base form or in pharmaceutically acceptable acid addition salt form.

It also includes the use of such a compound in the above indications

It also includes the compounds of formula I in free base form or in pharmaceutically acceptable acid addition salt form for use in the above indications.

The following Example, in which all temperatures are in °C, illustrates the invention:

## EXAMPLE

2,3-Dihydro-5-[4-[2-(3,4,5-trimethoxyphenyl)ethyl] phenyl]imidazo[1,2-a]thieno[2,3-c]pyridine

[R's = H; $R_2$ = 2-(3,4,5-trimethoxyphenyl)ethyl in the 4' position; T = a group (d) wherein $R_1$ is H]

To a solution of 2.8 g (6.1 mmol) of the compound prepared as described below in 100 ml of dry benzene was added 0.3 g of p-toluenesulfonic acid monohydrate and the resultant mixture was heated at reflux for 16 hours using a Dean-Stark trap to remove water. The reaction mixture was then cooled to room temperature, diluted with methylene chloride, washed successively with water, a saturated sodium bicarbonate solution and brine, dried over magnesium sulfate and filtered. The filtrate was then evaporated under reduced pressure and the crude product was purified by column chromatography on silica gel employing a mixture of methylene chloride and methanol in a 9:1 ratio as the eluent to yield the title compound in free

base form as a yellow foam.

Dry hydrochloride gas was bubbled into a solution of 0.4 g (0.9 mmol) of the above free base in a mixture of 20 ml of dry ethanol and 5 ml of methylene chloride for 5 minutes. The excess gas and the solvents were evaporated under reduced pressure and the crude residue was purified by crystallization from a mixture of methylene chloride and ether to yield the title compound in hydrochloride acid addition salt form (hemihydrate) as a tan solid (M.P. 240-242 °C).

The starting material is obtained as follows:

To a solution of 1.0 g (6.1 mmol) of 4,5-dihydro-2-(3-methylthien-2-yl)-1H-imidazole in a mixture of 40 ml of dry tetrahydrofuran and 1.69g (15 mmol) of N,N,N',N'-tetramethylethylenediamine was added, at -78 °C under a nitrogen atmosphere, 8.4ml of a 1.6M solution of N-butyl-lithium in hexane, and the resultant mixture was stirred at -78 °C for 15 minutes. To this mixture was added a solution of 2.0 g (6.1 mmol) of methyl-4-[2-(3,4,5-trimethoxyphenyl)ethyl]benzoate in 10ml of dry tetrahydrofuran and the reaction mixture was allowed to warm to ambient temperature and than stirred at ambient temperature for 2 hours. The mixture was then quenched with saturated ammonium chloride solution and extracted with methylene chloride. The combined organic extracts were then washed successively with water and brine, dried over magnesium sulfate and filtered. The filtrate was then evaporated under reduced pressure to yield an orange foam.

**Claims**

**Claims for the following Contracting States : DE, NL, AT, SE, CH, IT, BE, FR, LU, GB, LI**

**1.** A compound of formula I:

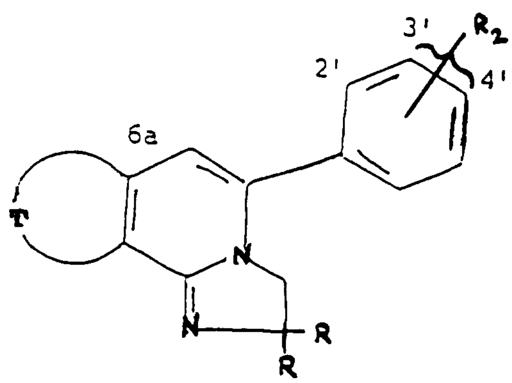

I

wherein

each R is, independently, hydrogen or methyl;

T is

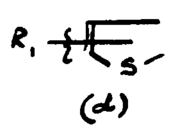

where $R_1$ is hydrogen or methyl; and

$R_2$ is a group of the formula

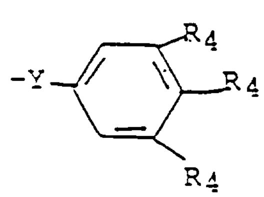

,

where
Y is $-(CH_2)_{1-3}$, $-OCH_2-$ or $-OCH_2CH_2-$; and each $R_4$, independently, is hydrogen or $C_{1-3}$alkoxy ;
in free base form or in acid addition salt form.

**2.** A compound according to claim 1 of formula Is:

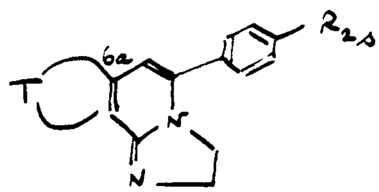

Is

wherein
T is as defined in claim 1 and
$R_{2s}$ is a group of the formula wherein

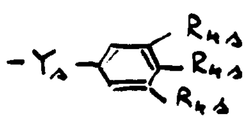

$Y_s$ is $-(CH_2)_{1-3}-$ and each $R_{4s}$ independently, is $C_{1-3}$alkoxy;
in free base form or in acid addition salt form.

**3.** The compound according to claim 1 where the R's are hydrogen, $R_2$ is 2-(3,4,5-trimethoxyphenyl)ethyl in the 4' position, and T is a group (d) wherein $R_1$ is hydrogen, in free base form or in acid addition salt form.

**4.** A pharmaceutical composition comprising a compound according to any one of claims 1 to 3 in free base form or in pharmaceutically acceptable acid addition salt form.

**5.** A compound according to any one of claims 1 to 3 in free base form or in pharmaceutically acceptable acid addition salt form, for use as a pharmaceutical.

**6.** A compound according to claim 5 for use in
- inhibiting PAF-mediated bronchoconstriction,
- inhibiting PAF-mediated extravasation,
- the control of hyperreactive airways induced by PAF or allergen,
- protecting against endotoxin-induced hypotension or
- protecting against endotoxin-induced death.

**7.** A compound according to claim 8 for use in inhibiting the growth of lymphomas, sarcomas, myelomas and leukemia cell lines.

**8.** A process for the preparation of a compound according to claim 1 in free base form or in acid addition salt form which comprises
dehydrating a corresponding compound of formula V

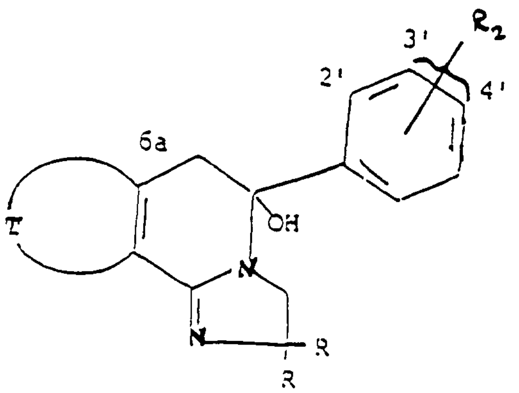

V

where the R's, T and $R_2$ are as defined in claim 1,
and recovering the resultant compound of formula I in free base form or in acid addition salt form.

**9.** A process for the preparation of a pharmaceutical composition which comprises mixing a compound of formula I as defined in claim 1 in free base form or in pharmaceutically acceptable acid addition salt form, with a pharmaceutically acceptable carrier or diluent.

**10.** Use of a compound of formula I as defined in claim 1 in free base form or in pharmaceutically acceptable acid addition salt form for the preparation of a pharmaceutical composition according to the process of claim 9.

**Claims for the following Contracting States : ES, GR**

**1.** A process for the preparation of a compound of formula I:

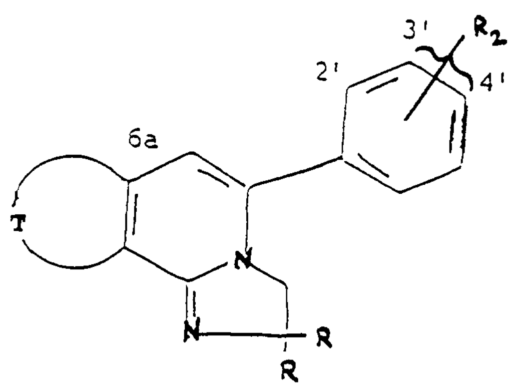

I

wherein
each R is, independently, hydrogen or methyl;
T is

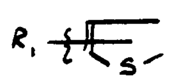

(d)

where $R_1$ is hydrogen or methyl; and
$R_2$ is a group of the formula

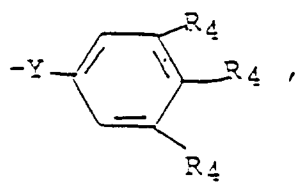

where

Y is $-(CH_2)_{1-3}$, $-OCH_2-$ or $-OCH_2CH_2-$; and each $R_4$, independently, is hydrogen or $C_{1-3}$alkoxy ;

in free base form or in acid addition salt form ,

which comprises dehydrating a corresponding compound of formula V

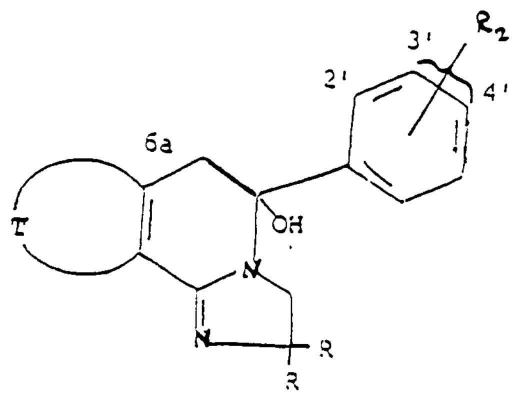

V

where the R's, T and $R_2$ are as defined in this claim,

and recovering the resultant compound of formula In in free base form or in acid addition salt form.

**2.** A process according to claim 1 for the preparation of a compound of formula Is:

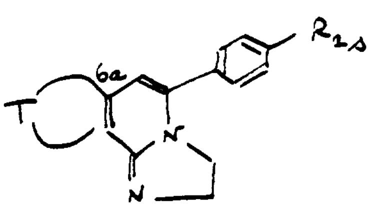

Is

wherein

T is as defined in claim 1 and

$R_{2s}$ is a group of the formula

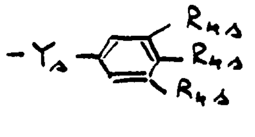

wherein $Y_s$ is $-(CH_2)_{1-3}-$ and each $R_{4s}$ independently, is $C_{1-3}$alkoxy;

in free base form or in acid addition salt form.

**3.** A process according to claim 1 for the preparation of the compound where the R's are hydrogen, $R_2$ is 2-(3,4,5-trimethoxyphenyl)ethyl in the 4' position, and T is a group (d) wherein $R_1$ is hydrogen, in free base form or in acid addition salt form.

4. A process for the preparation of a pharmaceutical composition which comprises mixing a compound of formula I as defined in claim 1 in free base form or in pharmaceutically acceptable acid addition salt form, with a pharmaceutically acceptable carrier or diluent.

**Patentansprüche**
**Patentansprüche für folgende Vertragsstaaten : DE, NL, AT, SE, CH, IT, BE, FR, LU, GB, LI**

1. Verbindung der Formel I

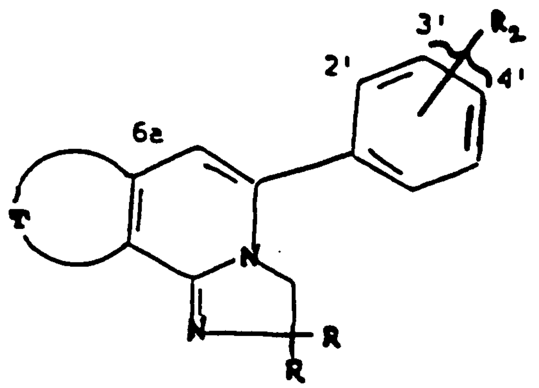

I

worin jedes R unabhängig für Wasserstoff oder Methyl steht,
T für

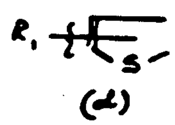

steht,
wobei $R_1$ für Wasserstoff oder Methyl steht, und
$R_2$ für eine Gruppe folgender Formel steht

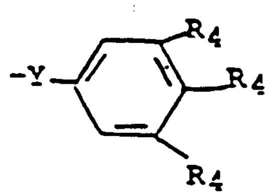

wobei Y für $-(CH_2)_{1-3}$, $-OCH_2-$ oder $-OCH_2CH_2-$ steht und jedes $R_4$ unabhängig für Wasserstoff oder $C_{1-3}$Alkoxy steht,
in Form der freien Base oder in Säureadditionssalzform.

2. Verbindung nach Anspruch 1 der Formel 1s

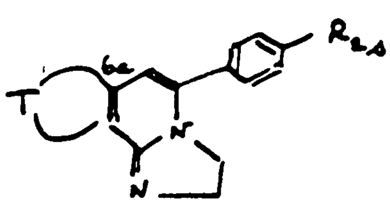

Is

worin T wie in Anspruch 1 definiert ist,
$R_{2s}$ für eine Gruppe folgender Formel steht

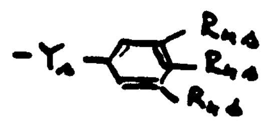

worin $Y_s$ für $-(CH_2)_{1-3}-$ steht und jedes $R_{4s}$ unabhängig für $C_{1-3}$Alkoxy steht, in Form der freien Base oder in Säureadditionssalzform.

3. Verbindung nach Anspruch 1, wobei die Reste R für Wasserstoff stehen, $R_2$ für 2-(3,4,5-trimethoxyphenyl)ethyl an der Position 4' steht und T für eine Gruppe (d) steht, worin $R_1$ für Wasserstoff steht, in Form einer freien Base oder in Säureadditionssalzform.

4. Pharmazeutische Zusammensetzung, die eine Verbindung nach einem der Anspüche 1 bis 3 umfaßt in Form der freien Base oder in pharmazeutisch annehmbarer Säureadditionssalzform.

5. Verbindung nach einem der Ansprüche 1 bis 3 in Form einer freien Base der in pharmazeutisch annehmbarer Säureadditionssalzform zur Verwendung als Pharmazeutikum.

6. Verbindung nach Anspruch 5 zur Verwendung bei
   - der Hemmung der PAF-vermittelten Bronchokonstriktion,
   - der Hemmung der PAF-vermittelten Extravasation,
   - der Kontrolle von hyperreaktiven Atemwegen, die durch PAF oder ein Allergen induziert werden,
   - dem Schutz gegen Endotoxin-induzierter Hypotension oder
   - dem Schutz gegen Endotoxin-induzierten Tod.

7. Verbindung nach Anspruch 8 zur Verwendung bei der Hemmung des Wachstums von Lymphomen, Sarcomen, Myelomen und Leukämiezellinien.

8. Verfahren zur Herstellung einer Verbindung nach Anspruch 1 in Form einer freien Base oder in Säureadditionssalzform, gekennzeichnet durch
Dehydrierung einer entsprechenden Verbindung der Formel V

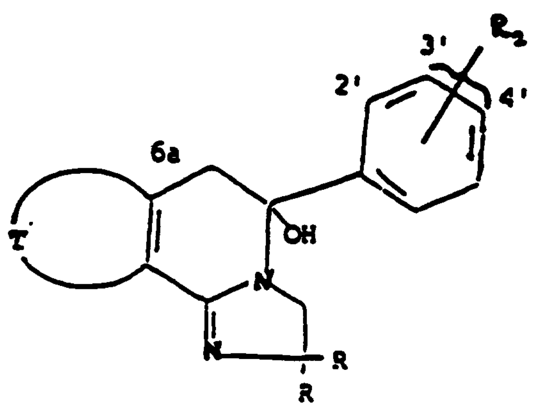

V

wobei die Reste R, T und $R_2$ wie in Anspruch 1 definiert sind,
und Gewinnung der entstehenden Verbindung der Formel I in Form der freien Base oder in Säureadditionssalzform.

9. Verfahren zur Herstellung einer pharmazeutischen Zusammensetzung, gekennzeichnet durch Mischen einer Verbindung der Formel I nach Anspruch 1 in Form der freien Base oder in pharmazeutisch annehmbarer Säureadditionssalzform mit einem pharmazeutisch annehmbaren Träger oder Verdünnungsmittel.

**10.** Verwendung einer Verbindung der Formel I nach Anspruch 1 in Form der freien Base oder in pharmazeutisch annehmbarer Säureadditionssalzform zur Herstellung einer pharmazeutischen Zusammensetzung gemäß dem Verfahren nach Anspruch 9.

**Patentansprüche für folgende Vertragsstaaten : ES, GR**

**1.** Verfahren zur Herstellung einer Verbindung der Formel I

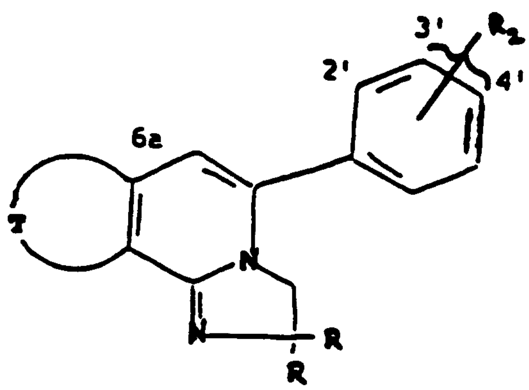

I

worin jedes R unabhängig für Wasserstoff oder Methyl steht,
T für

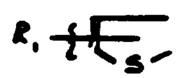

(α)

steht,
wobei $R_1$ für Wasserstoff oder Methyl steht, und
$R_2$ für eine Gruppe folgender Formel steht

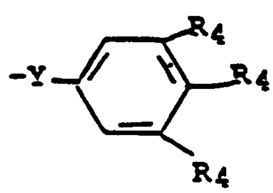

wobei Y für $-(CH_2)_{1-3}$, $-OCH_2-$ oder $-OCH_2CH_2-$ steht und jedes $R_4$ unabhängig für Wasserstoff oder $C_{1-3}$Alkoxy steht,
in Form der freien Base oder in Säureadditionssalzform,
gekennzeichnet durch Dehydrierung einer entsprechenden Verbindung der Formel V

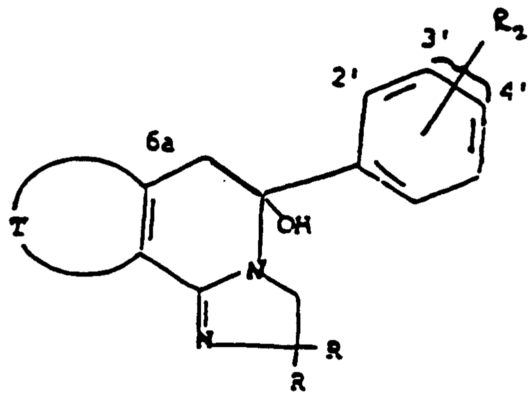

V

wobei die Reste R, T und $R_2$ wie in diesem Anspruch definiert sind,
und Gewinnung der entstehenden Verbindung der Formel I in Form der freien Base oder in Säureadditionssalzform.

2. Verfahren nach Anspruch 1 zur Herstellung einer Verbindung der Formel Is

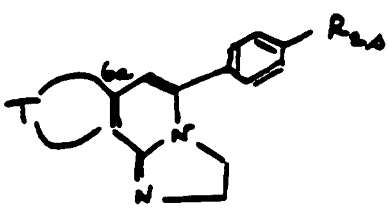

Is

worin T wie in Anspruch 1 definiert ist, und
$R_{2s}$ für eine Gruppe folgender Formel steht

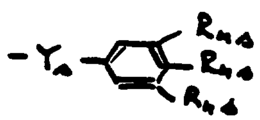

worin $Y_s$ für $-(CH_2)_{1-3}-$ steht und jedes $R_{4s}$ unabhängig für $C_{1-3}$Alkoxy steht,
in Form der freien Base oder in Säureadditionssalzform.

3. Verfahren nach Anspruch 1 zur Herstellung der Verbindung, in der die Reste R für Wasserstoff stehen, $R_2$ für 2-(3,4,5-Trimethoxyphenyl)ethyl an der Position 4' steht und T für eine Gruppe (d) steht, worin $R_1$ für Wasserstoff steht,
in Form einer freien Base oder in Säureadditionssalzform.

4. Verfahren zur Herstellung einer pharmazeutischen Zusammensetzung, gekennzeichnet durch Mischen einer Verbindung der Formel I nach Anspruch 1 in Form der freien Base oder in pharmazeutisch annehmbarer Säureadditionssalzform mit einem pharmazeutisch annehmbaren Träger oder Verdünnungsmittel.

**Revendications**

**Revendications pour les Etats contractants suivants : DE, NL, AT, SE, CH, IT, BE, FR, LU, GB, LI**

**1.** Un composé de formule I

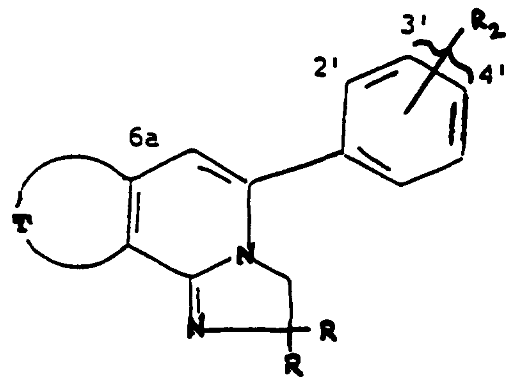

I

dans laquelle

| | |
|---|---|
| chaque R | signifie indépendamment l'hydrogène ou un groupe méthyle, |
| T | signifie |

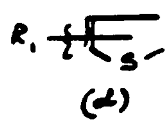

où $R_1$ signifie l'hydrogène ou un groupe méthyle, et

| | |
|---|---|
| $R_2$ | signifie un groupe de formule |

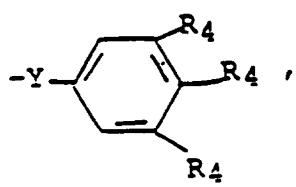

où Y signifie $-(CH_2)_{1-3}$, $-OCH_2-$ ou $-OCH_2CH_2-$, et chaque $R_4$ signifie indépendamment l'hydrogène ou un groupe alcoxy en $C_1$-$C_3$,

sous forme de base libre ou d'un sel d'addition d'acide.

**2.** Un composé selon la revendication 1 de formule Is

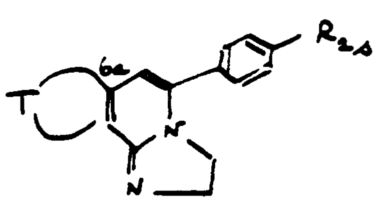

Is

dans laquelle

T est tel que défini à la revendication 1, et

$R_{2a}$ signifie un groupe de formule

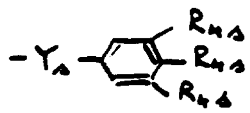

dans laquelle $Y_s$ signifie $-(CH_2)_{1-3}-$ et chaque $R_{4s}$ signifie indépendamment un groupe alcoxy en $C_1$-$C_3$,

sous forme de base libre ou d'un sel d'addition d'acide.

**3.** Le composé selon la revendication 1 où les symboles R signifient l' hydrogène, $R_2$ signifie un groupe 2-(3,4,5-triméthoxyphényl)éthyle en position 4', et T signifie un groupe (d) dans lequel $R_1$ signifie l'hydrogène,
sous forme de base libre ou d'un sel d'addition d'acide.

**4.** Une composition pharmaceutique comprenant un composé selon l'une quelconque des revendications 1 à 3, sous forme de base libre ou d'un sel d'addition d' acide pharmaceutiquement acceptable.

**5.** Un composé selon l'une quelconque des revendications 1 à 3, sous forme de base libre ou d'un sel d'addition d'acide pharmaceutiquement acceptable, pour l'utilisation comme médicament.

**6.** Un composé selon la revendication 5 pour l'utilisation dans
- l'inhibition de la bronchoconstriction induite par le PAF,
- l'inhibition de l'extravasation induite par le PAF,
- le contrôle de l'hyperréactivité des voies respiratoires induite par le PAF ou un allergène,
- la protection contre l'hypotension induite par les endotoxines ou bien
- la protection contre le décès provoqué par les endotoxines.

**7.** Un composé selon la revendication 8, pour l'utilisation dans l'inhibition de la croissance des lymphomes, sarcomes, myélomes et des lignées cellulaires de la leucémie.

**8.** Un procédé de préparation d'un composé selon la revendication 1, sous forme de base libre ou d'un sel d'addition d'acide, lequel procédé comprend la déshydratatation d'un composé correspondant de formule V

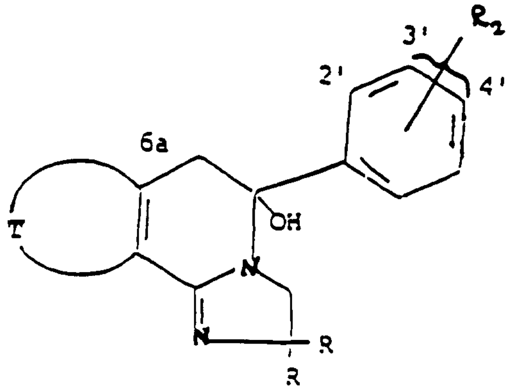

V

dans laquelle les symboles R, T et $R_2$ sont tels que définis à la revendication 1, et la résupération du composé résultant de formule I sous forme de base libre ou d'un sel d'addition d' acide.

**9.** Un procédé de préparation d'une composition pharmaceutique, comprenant le mélange d'un composé de formule I tel que défini à la revendication 1, sous forme de base libre ou d'un sel d'addition d'acide pharmaceutiquement acceptable, avec un véhicule ou un diluant pharmaceutiquement acceptable.

**10.** L'utilisation d'un composé de formule I tel que défini à la revendication 1, sous forme de base libre ou d'un sel d'addition d'acide pharmaceutiquement acceptable, pour la préparation d'une composition pharmaceutique selon le procédé de la revendication 9.

**Revendications pour les Etats contractants suivants : ES, GR**

**1.** Un procédé de préparation d'un composé de formule I

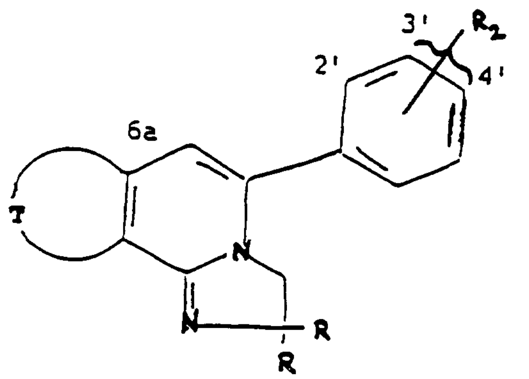

I

dans laquelle

chaque R signifie indépendamment l'hydrogène ou un groupe méthyle,

T signifie

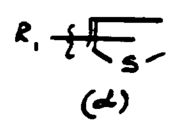

où $R_1$ signifie l'hydrogène ou un groupe méthyle, et

$R_2$ signifie un groupe de formule

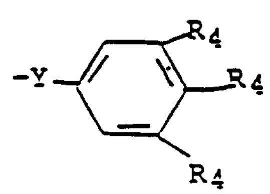 ,

où Y signifie $-(CH_2)_{1-3}$, $-OCH_2-$ ou $-OCH_2CH_2-$, et chaque $R_4$ signifie indépendamment l'hydrogène ou un groupe alcoxy en $C_1$-$C_3$,

sous forme de base libre ou d'un sel d'addition d'acide,

lequel procédé comprend

la déshydratation d'un composé correspondant de formule V

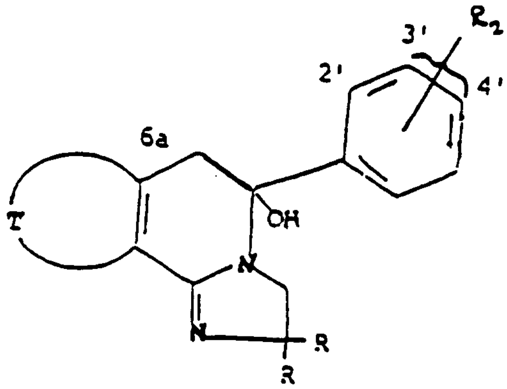

V

dans laquelle les symboles R, T et $R_2$ sont tels que définis à la présente revendication, et la récupération du composé résultant de formule I sous forme de base libre ou d'un sel d'addition d'acide.

2. Un procédé selon la revendication 1 pour la préparation d'un composé de formule Is

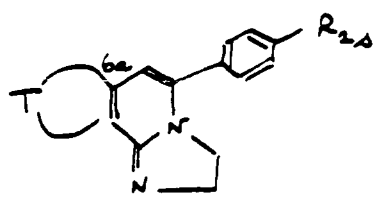

Is

dans laquelle
T est tel que défini à la revendication 1, et
$R_{2a}$ signifie un groupe de formule

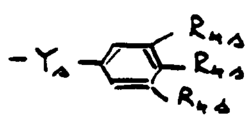

dans laquelle $Y_s$ signifie $-(CH_2)_{1-3}-$ et chaque $R_{4s}$ signifie indépendamment un groupe alcoxy en $C_1$-$C_3$,
sous forme de base libre ou d'un sel d'addition d'acide.

3. Un procédé selon la revendication 1 pour la préparation du composé dans lequel les symboles R signifient l'hydrogène, $R_2$ signifie un groupe 2-(3,4,5-triméthoxyphényl)éthyle en position 4', et T signifie un groupe (d) dans lequel $R_1$ signifie l'hydrogène, sous forme de base libre ou d'un sel d'addition d'acide.

4. Un procédé de préparation d'une composition pharmaceutique, comprenant le mélange d'un composé de formule I tel que défini à la revendication 1, sous forme de base libre ou d'un sel d'addition d'acide pharmaceutiquement acceptable, avec un véhicule ou un diluant pharmaceutiquement acceptable.